(19)

Europäisches
Patentamt
European
Patent Office
Office européen
des brevets

(11) **EP 3 207 855 A1**

(12) **EUROPEAN PATENT APPLICATION**
published in accordance with Art. 153(4) EPC

(43) Date of publication:
**23.08.2017 Bulletin 2017/34**

(51) Int Cl.:
*A61B 1/04* *(2006.01)*　　　*G02B 23/24* *(2006.01)*

(21) Application number: **15850738.4**

(86) International application number:
**PCT/JP2015/077981**

(22) Date of filing: **01.10.2015**

(87) International publication number:
**WO 2016/059977 (21.04.2016 Gazette 2016/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **15.10.2014 JP 2014211035**

(71) Applicant: **Olympus Corporation
Tokyo 192-8507 (JP)**

(72) Inventor: **SATO, Tomoya
Hachioji-shi
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(54) **SIGNAL PROCESSING DEVICE AND ENDOSCOPE SYSTEM**

(57)　　A signal process apparatus (4) includes a contour extraction unit (441) that acquires a plurality of image data items generated by sequential imaging of a subject, and extracts contour components from the acquired plurality of image data items; a shift amount detector (443) that compares shift amounts of contour components extracted by the contour extraction unit between image data items included in each of a plurality of image sets, each of the image sets having a predetermined number of the image data items, and detects a shift amount between image data items included in each of the image sets; and an image selection unit (444) that selects at least one image set among the image sets based on a detection result of the shift amount detector.

FIG.4

**Description**

Technical Field

The invention relates to a signal process apparatus that processes an image input from an endoscope apparatus, and an endoscope system comprising the same.

Background Art

For an endoscope apparatus having an electronic endoscope apparatus, a function of generating and displaying a still image from a video endoscope image has been known. Such a still image is generated by using image data of a plurality of fields. Accordingly, if a subject or an endoscope apparatus moves, a still image with a large color-shift is generated. For example, Jpn. Pat. Appln. KOKAI Publication No. 2006-149483 provides a suggestion for avoiding color-shift when displaying a still image. Jpn. Pat. Appln. KOKAI Publication No. 2006-149483 discloses an electronic endoscope apparatus that determines a degree of color-shift, which is a shift between images of different colors, based on the symbol of difference in pixel values between adjacent pixels in the same horizontal line of image data, each having a different color obtained by the field sequential method, i.e., the change in pixel value. The electronic endoscope apparatus of Jpn. Pat. Appln. KOKAI Publication No. 2006-149483 corrects a color-shift by shifting image data so that the color-shift is minimized.

Summary of Invention

If a color-shift is determined only based on the way of a change in luminance, a change in pixel value due to noise, for example, may be determined as a color-shift. In addition, an image having halation or an overexposed image that is not suitable for still image display may be determined as an image not having a color-shift.
The present invention is made in consideration of the above circumstances, and the object of the present invention is to provide a signal process apparatus capable of generating a still image without being affected by noise or the brightness of a subject itself when generating a still image from an image input from an endoscope apparatus, and an endoscope system comprising the same.
To achieve the object, a signal process apparatus according to a first aspect of the invention comprises: a contour extraction unit that acquires a plurality of image data items generated by sequential imaging of a subject, and extracts contour components from the acquired plurality of image data items; a shift amount detector that compares shift amounts of contour components extracted by the contour extraction unit between image data items included in each of a plurality of image sets, each of the image sets having a predetermined number of the image data items, and detects a shift amount between image data items included in each of the image sets; and an image selection unit that selects at least one image set among the image sets based on a detection result of the shift amount detector.
To achieve the object, an endoscope system according to a second aspect of the invention comprises: an imaging unit that acquires a plurality of image data items by sequential imaging of a subject, a; a contour extraction unit that extracts contour components from the plurality of image data items acquired at the imaging unit; a shift amount detector that compares shift amounts of contour components extracted by the contour extraction unit between image data items included in each of a plurality of image sets, each of the image sets having a predetermined number of the image data items, and detects a shift amount between image data items included in each of the image sets; and an image selection unit that selects at least one image set among the image sets based on a detection result of the shift amount detector.
The present invention is to provide a signal process apparatus capable of generating a still image without being affected by noise or the brightness of a subject itself when generating a still image from an image input from an endoscope apparatus, and an endoscope system comprising the same.

Brief Description of Drawings

FIG. 1 is a block diagram showing the configuration of an endoscope system comprising a signal process apparatus according to an embodiment;
FIG. 2 is a flowchart showing the display processing of an endoscope image by the endoscope system;
FIG. 3 is a flowchart showing the processing of selecting an image set;
FIG. 4 illustrates an example of selection of an image set in accordance with the processing shown in FIG. 3;
FIG. 5 illustrates the configuration of a still image selection unit of a modification;

FIG. 6 is a flowchart showing the processing of selecting an image set of a modification.

Description of Embodiments

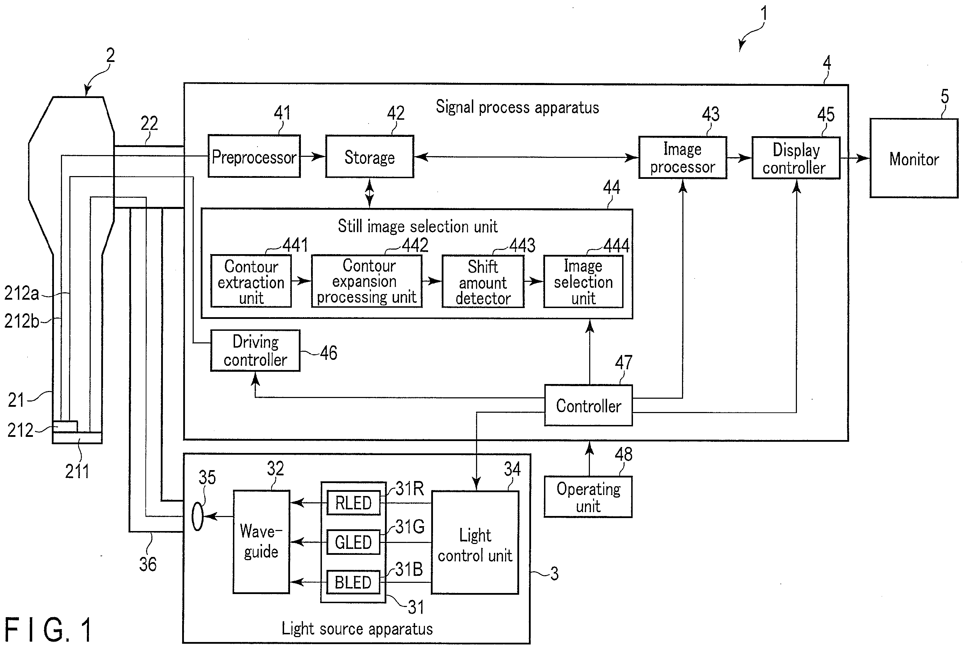

Hereinafter, embodiments of the present invention will be described with reference to the drawings. FIG. 1 is a block diagram showing the configuration of an endoscope system comprising a signal process apparatus according to an embodiment. An endoscope system 1 shown in FIG. 1 is a system that generates an endoscope image by the field sequential method, for example. The field sequential method is a method of obtaining a colored endoscope image by irradiating a subject with a different color of illumination light for each field, and synchronizing signals obtained in the field-sequential method from an imager.

An endoscope system 1 includes an endoscope apparatus 2, a light source apparatus 3, a signal process apparatus 4 and a monitor 5. The endoscope apparatus 2 images inside a body of a subject and generates an image signal for the subject. The light source apparatus 3 is connected to the endoscope apparatus 2 through a light guide 36, and supplies illumination light to the endoscope apparatus 2. The signal process apparatus 4 processes an image signal generated at the endoscope apparatus 2. The monitor 5 displays an endoscope image which is an image of the inside of the body of the subject, for example, based on the image signal processed at the signal process apparatus 4. In the following, each block of the endoscope system 1 will be explained.

The endoscope apparatus 2 includes an insertion portion 21 and a cable 22. The insertion portion 21 is a portion of the endoscope apparatus 2 which is to be inserted inside the body of the subject. The insertion portion 21 is provided with an objective lens 211 and an imager 212 at a distal end. The objective lens 211 is an optical system to form an image from the subject at the imager 212. The imager 212 has two-dimensionally arranged pixels. Each pixel is a photo diode, for example, and generates an electrical signal (image signal) in accordance with the amount of applied light through the objective lens 211. As mentioned above, the endoscope system 1 generates an endoscope image by the field sequential method. Accordingly, the imager 212 performs imaging multiple times during the period of displaying one frame of an endoscope image. In each of the multiple times of imaging, an illumination light of a different color is applied to the subject. The imager 212 generates an image signal having only information of one of the different colors for each time of imaging. In the embodiment, a red (R) illumination light, a green (G) illumination light, and a blue (B) illumination light are sequentially applied. If the R illumination light is applied to the subject, an image signal having only information of R is generated, if the G illumination light is applied to the subject, an image signal having only information of G is generated, and if the B illumination light is applied to the subject, an image signal having only information of B is generated.

The cable 22 is connected to the signal process apparatus 4 through a connector not shown in the drawings. A drive signal line 212a and an output signal line 212b are provided inside of the cable 22. The drive signal line 212a is connected to each pixel of the imager 212. The drive signal line 212a is arranged to be connected to a drive controller 46 of the signal process apparatus 4 when the insertion portion 21 is connected to the signal process apparatus 4 through a connector not shown in the drawings. In this case, the imager 212 performs imaging of the subject in accordance with a drive signal from the drive controller 46 through the drive signal line 212a. The output signal line 212b is also connected to each pixel of the imager 212. The output signal line 212b is arranged to be connected to a preprocessor 41 of the signal process apparatus 4 when the insertion portion 21 is connected to the signal process apparatus 4 through the connector not shown in the drawings. In this case, the imager 212 outputs an image signal to the preprocessor 41 through the output signal line 212b.

The light source apparatus 3 includes a light source 31, a waveguide 32, a light control unit 34, a condenser lens 35, and the light guide 36.

The light source 31 includes a plurality of light sources such as RLED31R, GLED31G, and BLED31B. RLED31R is an LED that emits a red (R) light beam. GLED31G is an LED that emits a green (G) light beam. BLED31B is an LED that emits a blue (B) light beam. The waveguide 32 is, for example, an optical fiber which is optically coupled to each of RLED31R, GLED31G, and BLED31B. The waveguide 32 guides an illumination light beam emitted from each of RLED31R, GLED31G, and BLED31B to the condenser lens 35. The light control unit 34 controls the operation of RLED31R, GLED31G, and BLED31B synchronized with the imaging operation of the imager 212 controlled by the drive controller 46. In the field sequential method, the light control unit 34 sequentially drives RLED31R, GLED31G, and BLED31B for each field so that RLED31R, GLED31G, and BLED31B emit a light beam in a field-sequential manner. The condenser lens 35 condenses a light beam emitted from the waveguide 32 and applies the condensed light beam to the light guide 36. The light guide 36 applies the light beam condensed by the condenser lens 35 to the endoscope apparatus 2. The light beam is emitted from the endoscope apparatus 2 and applied to the subject. The subject is imaged in this manner, and each of an R image signal, a G image signal and a B image signal is obtained at the imager 212. The light source apparatus 3 may be provided with a white light source (a lamp or a white LED) and an RGB rotation filter so that the field sequential method is accomplished by sequentially inserting the RGB filter to the light path for each field, instead of providing with RLED, GLED, and BLED. The light source 31 may be capable of allowing the RLED31R, GLED31G, and BLED31B to emit light simultaneously, or emitting visible light directly from the white light source, to emit white light. This illumination method is called a simultaneous-type method, for example.

The signal process apparatus 4 includes the preprocessor 41, a storage 42, an image processor 43, a still image selection unit 44, a display controller 45, a display controller 46, a controller 47, and an operating unit 48.

The preprocessor 41 performs various kinds of analog processing such as analog gain adjustment, and analog noise elimination of an image signal. In addition, the preprocessor 41 also converts an image signal subjected to analog processing into image data which is a digital signal. In the following explanation, a set of image data items each having a different color obtained by multiple imagings by the imager 212 is referred to as an image set. The image set is a set of multiple items of image data used for generating one item of a color endoscope image. In the present embodiment, an image data set is composed of three items of image data. That is, the three items of image data are R image data having only red (R) information, G image data having only green (G) information, and B image data having only blue (B) information.

For the endoscope system, it is desirable that the endoscope apparatus 2 and the signal process apparatus 4 are insulated by an insulation circuit. The insulation circuit is provided, for example, at the preprocessor 41. The insulation circuit includes a circuit that wirelessly transmits a signal, such as a photocoupler, for example.

The storage 42 temporarily stores image data obtained by the preprocessor 41. The storage 42 has a storage capacity required for storing multiple image sets.

The image processor 43 performs image processing to each image data item included in an image set stored in the storage 42. The image processor 43 performs image processing for video display to an input image data when displaying video, and performs image processing for still image display to an input image data when displaying a still image. The image processor 43 includes a synchronization circuit, a luminance/color difference isolation circuit, a gradation correction circuit, a color correction circuit, a scaling circuit, and an enhancement circuit, for example. The synchronization circuit synchronizes three items of image data included in an image set obtained in accordance with the field sequential method to generate a synchronized image data. The synchronized image data is image data in which each pixel has information of multiple types of color components. The gradation correction circuit performs gradation correction to the synchronized image data. The color correction circuit performs color correction to the synchronized image data. The scaling circuit enlarges or reduces the size of the synchronized image data in accordance with the display size of the monitor 5 to generate a display image data. The enhancement circuit performs processing to enhance the contour of the subject in the display image data, or to enhance the structure of a blood vessel, for example, when the subject is a blood vessel.

The still image selection unit 44 performs image selection for still image display by using image data included in an image set stored in the storage 42. The still image selection unit 44 includes a contour extraction unit 441, a contour expansion processing unit 442, a shift amount detector 443, and an image selection unit 444.

The contour extraction unit 441 includes a contour extraction filter such as a Laplacian filter or a Sobel filter, and extracts a contour component (a high-frequency component having a predetermined value or higher) of each image data item included in an image set stored in the storage 42. The contour extraction processing by the contour extraction unit 441 may be performed by signal processing on the frequency domain using a Fourier transform (FFT), for example, instead of filter processing. The contour expansion processing unit 442 performs processing to expand the contour component extracted by the contour extraction unit 441. The contour expansion processing is performed by thickening an extracted contour line, or blurring using a smoothing filter such as a Gaussian filter. The contour extraction and smoothing may be performed by a single filter.

The shift amount detector 443 detects a shift amount of contour components extracted from image data items included in an image set as a shift amount between image data items included in the image set. The image selection unit 444 selects an image set to be used for still image display based on the shift amount detected by the shift amount detector 443. That is, the image selection unit 444 selects as an image set for still image display an image set having the smallest shift amount between image data items among multiple image sets obtained by imaging for video display. Image data included in the image set selected at the image selection unit 444 is obtained from the storage 42 by the image processor 43.

The display controller 45 displays on the monitor 5 an endoscope image colored based on the image data (display image data) subjected to the image processing at the image processor 43. The display controller 45 converts the display image data which is a digital signal to an analog video signal, for example. In addition, the display controller 45 inputs a video signal to the monitor 5 in response to an instruction from the controller 47 to display the endoscope image to the monitor 5. The drive controller 46 controls the imaging operation of the imager 212. That is, the drive controller 46 controls exposure initiation or termination of the imager 212 and controls reading of an image signal from the imager 212.

The controller 47 controls the entire operation of the endoscope system 1 including the operation of the signal process apparatus 4. For example, the controller 47 synchronizes control of the light source 31 by the light control unit 34 with imaging control of the imager 212 by the drive controller 46. The controller 47 also controls the display controller 45. In addition, the controller 47 causes the still image selection unit 44 to perform processing for still image display in response to a still image display instruction through the operating unit 48.

The operating unit 48 includes an operating member through which a user operates the endoscope system 1. In the present embodiment, the operating unit 48 includes an operating member by which the user instructs still image display of an endoscope image. The operating member may be a button, a switch, or a touch panel.

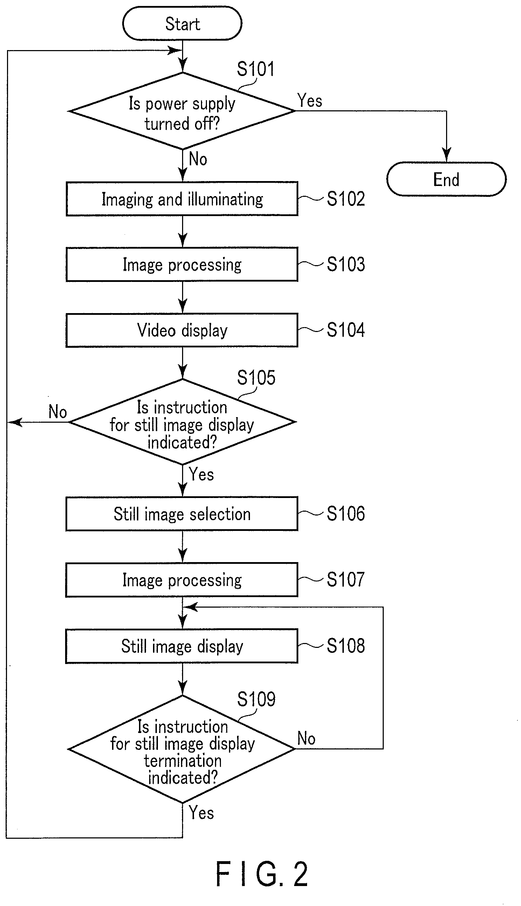

The monitor 5 is a monitor such as a liquid crystal display, and displays various types of images such as an endoscope image, in accordance with control by the display controller 45. Next, the operation of the endoscope system 1 according to the present embodiment will be explained. FIG. 2 is a flowchart showing the display processing of an endoscope image by the endoscope system 1. The processing shown in FIG. 2 is initiated when the power supply of the endoscope system 1 is turned on, for example.

In step S101, the controller 47 determines whether or not the power supply of the endoscope system 1 is turned off. In step S101, if it is determined that the power supply of the endoscope system 1 is turned off, the operation shown in FIG. 2 is terminated.

In step S101, if it is determined that the power supply of the endoscope system 1 is not turned off, the processing proceeds to step S102. In step S102, the controller 47 causes the drive controller 46 to initiate imaging control of the imager 212, and causes the light source apparatus 3 to start light emission so as to be synchronized with imaging of the imager 212. The light control unit 34 controls the light source 31 so that the light source apparatus 3 emits a different color of light for each predetermined field period, for example. On the other hand, the drive controller 46 controls the imager 212 so that imaging is performed in each predetermined field period. The image data is stored in the storage 42 in the aforementioned processing.

In step S103, the controller 47 causes the image processor 43 to perform image processing for video display of an endoscope image. The image processor 43 performs basic processing for video display such as synchronization, gradation correction, color correction, scaling, and enhancement of image data.

In step S104, the controller 47 causes the display controller 45 to display an endoscope image based on the image data processed at the image processor 43. That is, the display controller 45 converts display image data generated for every three fields (one frame) to a video signal and inputs the video signal to the monitor 5. The monitor 5 displays an endoscope image based on the video signal. The user can observe the inside of the body of the subject, for example, as a video by viewing the endoscope image displayed on the monitor 5.

In step S105, the controller 47 determines whether an instruction for still image display of an endoscope image which is being displayed as video is made by a user operation of the operating unit 48. When it is determined that no instruction for still image display is made in step S105, the processing returns to step S101. In this case, the video display of the endoscope image is continued.

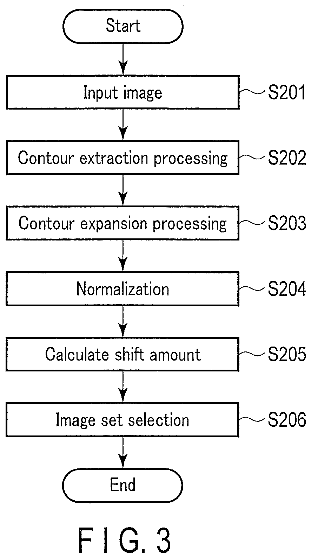

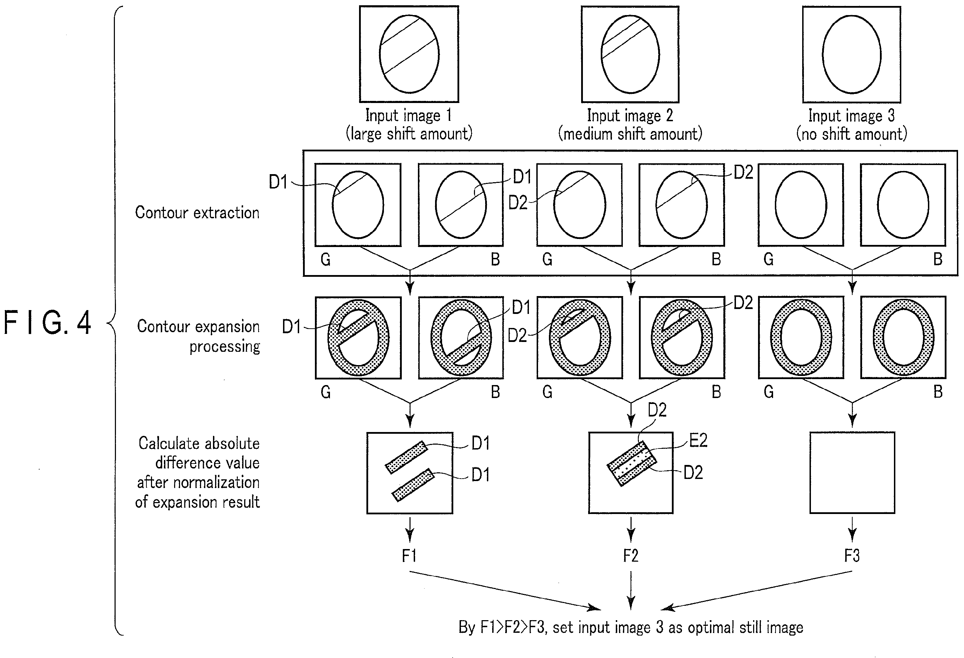

When it is determined that an instruction for still image display is made in step S105, the processing proceeds to step S106. In step S106, the controller 47 causes the still image selection unit 44 to select an image set for still image display. The operation of selecting an image set will be explained below. FIG. 3 is a flowchart showing the processing of selecting an image set. FIG. 4 illustrates an example of selection of an image set in accordance with the processing shown in FIG. 3. In step S201, the contour extraction unit 441 of the still image selection unit 44 acquires multiple image sets (each including R image data, G image data, and B image data) stored in the storage 42. In FIG. 4, three image sets including input image 1, input image 2, and input image 3 are acquired. The input image 1 is an image set in which a large amount of shift is generated between image data items. The input image 2 is an image set in which a medium amount of shift is generated between image data items. The input image 3 is an image set in which a medium amount of shift is generated (no shift amount is generated) between image data items.

In FIG. 4, an example of acquiring three image sets is shown. However, the number of image sets to be acquired can be suitably set, for example, by acquiring image sets until 500 milliseconds before the still image display instruction.

In addition, if, for example, a special light is emitted from the light source apparatus 3, the influence of G image data and B image data greatly affects display image data generated from the image set. Accordingly, the contour extraction unit 441 may be arranged to acquire only image data of two particular colors when it is anticipated that the particular colors greatly affect an image set. In the following explanation, it is assumed that the contour extraction unit 441 acquires only G image data and B image data, for example.

In step S202, the contour extraction unit 441 extracts contour components of G image data and B image data included in each of acquired image sets, for example, by a filtering processing. As shown in FIG. 4, if there is no color-shift between image data, there is no shift between the contours of image data extracted by contour extraction. On the other hand, if there is a color-shift between image data, contour shifts indicated by D1 or D2 occur between image data items. Accordingly, the shift amount between image data items can be detected based on the contour shift amount.

In step S203, the contour expansion processing unit 442 performs contour expansion processing to the contour components of the G image data and B image data extracted by the contour extraction unit 441. The magnitude of contour expansion processing may vary in accordance with the allowed amount of shifts between image data items. That is, if the allowed amount of shifts between image data items is large, the magnitude of contour expansion processing can be increased, and if the allowed amount of shifts between image data items is small, the magnitude of contour expansion processing needs to be decreased.

In step S204, the shift amount detector 443 normalizes contour components of R image data, G image data and B image data subjected to contour expansion processing at the contour expansion processing unit 442. The shift amount detector 443 normalizes the contour components by a maximum value that each pixel data can take in image data, for example.

In step S205, the shift amount detector 443 calculates a shift amount between image data included in the same image set. If a shift amount is F, the shift amount F is calculated by equation (1), i.e., L2norm (sum of squared differences) of each pixel of image data. In the equation (1), "I1 (x, y) " represents a pixel value of a position (x, y) in image data I1, which is one of R image data, G image data, and B image data after normalization, "I2 (x, y)" represents a pixel value of a position (x, y) in image data I2, which is another one of R image data, G image data, and B image data after normalization, "x" represents a horizontal position, and "y" represents a vertical position. For example, the left end position of image data is x=1, and the right end position of the image data is x=N. In addition, the top end position of image data is y=1, and the bottom end position of the image data is y=M.

$$F = \sum_{x=1}^{N} \sum_{y=1}^{M} \left( |I1(x, y) - I2(x, y)|^2 \right) \qquad \text{Equation (1)}$$

As shown in FIG. 4, the color-shift amount of image data items can be obtained as the contour shift amount by applying equation (1) to the image data after contour extraction. In particular, by performing contour expansion processing prior to obtaining a difference, overlapping portion E2 between contours is generated if the color-shift amount is small (medium level) between image data items. The overlapping portion E2 is eliminated by contour expansion processing. Accordingly, the result of equation (1) for the case of a medium level of color-shift amount becomes smaller than the result of equation (1) for the case of a large color-shift amount. Thus, as the color-shift amount becomes greater, the result of equation (1) becomes greater, and the color-shift amount between image data items can be suitably evaluated.

The calculation of equation (1) is usually performed between R image data and G image data; G image data and B image data; and B image data and R image data. However, if the contour components are extracted only for image data items of two particular colors, equation (1) is applied to the image data items.

In step S206, the image selection unit 444 selects an image set to be used for still image display, in accordance with the shift amounts between image data items included in each of the image sets. When applying equation (1), as the value of F is smaller, the contour shift amount becomes smaller. Thus, the image selection unit 444 selects an image set having the smallest shift amount between image data items as an image set for still image display. In the example of FIG. 4, the image selection unit 444 selects input image 3 as the image set to be used for still image display. After the aforementioned selection of the image set, the processing of FIG. 3 is terminated.

The explanation returns to FIG. 2. In step S107, the controller 47 causes the image processor 43 to perform image processing for still image display of an endoscope image by using the selected image set. The image processor 43 performs basic processing for still image display such as synchronization, gradation correction, color correction, scaling, and enhancement of image data.

In step S108, the controller 47 causes the display controller 45 to display an endoscope image based on the image data processed at the image processor 43. That is, the display controller 45 converts display image data generated by image processing for still image display to a video signal, and inputs the video signal to the monitor 5. The monitor 5 displays an endoscope image based on the video signal. The user can observe a still image of the inside of the body at necessary timings by viewing the endoscope image displayed on the monitor 5. In step S109, the controller 47 determines whether an instruction for termination of still image display is made by user operation of the operating unit 48. When it is determined that no instruction for still image display termination is made in step S109, the processing returns to step S108. In this case, the still image display of the endoscope image is continued.

When it is determined that an instruction for still image display termination is made in step S109, the processing returns to step S101. In this case, the video display is resumed if the power supply of the endoscope system 1 is not turned off. As explained above, in the present embodiment, the shift amount between image data items included in an image set is detected based on the shift amount of contour components of image data items included in the image set. Since contour components are not extracted from image data having a uniformly high luminance such as an image data having halation or an overexposed image data, the possibility of selecting an image set that is not suitable for still image display can be decreased. In addition, by calculating the shift amount F by L2norm of pixel value, not only the way of change in luminance in pixel, but also the sum of a pixel change amount can be evaluated. This improves noise resistance.

Furthermore, when calculating the shift amount F, blurring is performed to the contour components. Accordingly the noise resistance can be further improved. Blurring is not an essential processing, and may be performed in accordance with the allowed shift amount.

In this embodiment, an image set includes three items of image data obtained by the field sequential method. However, the image set is not limited to include image data items obtained by the field sequential method. For example, the image set may include image data obtained for each field of simultaneous-type method (interlace method). In the interlace method, different rows of pixels are exposed for each field, and accordingly, a shift occurs between image data items. The shift amount between image data items is detected by the technique of the present embodiment.

According to the present embodiment, the shift amount F is calculated by L2norm of pixel values. The shift amount F is not limited to L2norm of pixel values. For example, the shift amount F may be L1norm (sum of absolute difference values) of pixel values.

According to the present embodiment, one image set is selected in accordance with the shift amount F. However, the image selection unit 444 may be arranged to select multiple image sets having small shift amounts F.

[Variation Example]

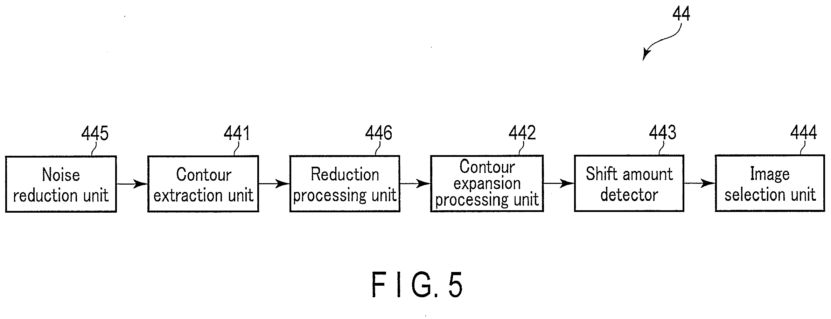

The variation example of the embodiment will be explained below. FIG. 5 illustrates the configuration of a still image selection unit 44 of a variation example. The still image selection unit 44 of the variation example includes a noise reduction unit 445, a contour extraction unit 441, a reduction processing unit 446, a contour expansion processing unit 442, a shift amount detector 443, and an image selection unit 444. The contour extraction unit 441, the contour expansion processing unit 442, the shift amount detector 443, and the image selection unit 444 have the same structures as explained with reference to FIG. 1. Accordingly, the explanation thereof will be omitted.

The noise reduction unit 445 is provided as a preprocessing stage of the contour extraction unit 441, and reduces noise components (components regarded as noise of frequencies higher than contour components) of each image data item included in image sets stored in the storage 42. By reducing noise components prior to the contour extraction processing by the contour extraction unit 441, a contour component can be extracted with high accuracy in the contour extraction processing. The reduction processing unit 446 is provided as a preprocessing stage of the contour expansion processing unit, for example, and performs reduction processing to the contour component of an image data item extracted at the contour extraction unit 441. The reduction processing is performed, for example by thinning or pixel addition processing. By reduction processing, the filter size required for contour expansion processing at the contour expansion processing unit 442 can be reduced, and the load of the contour expansion processing also can be reduced. The reduction processing unit 446 may be provided as a preprocessing stage of the contour extraction unit 441. In this case, the filter size required for contour extraction processing at the contour extraction unit 441 can be reduced, and the load of the contour extraction processing also can be reduced.

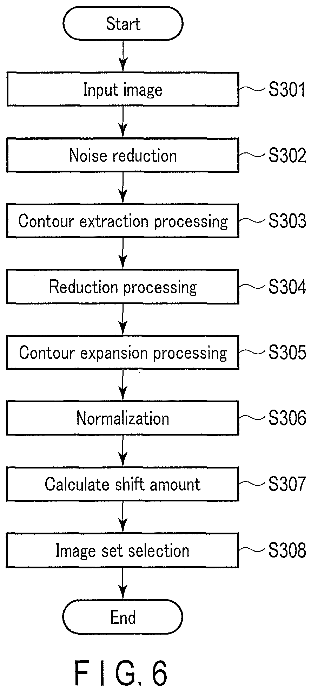

FIG. 6 is a flowchart showing the processing of selecting an image set in the variation example. The same processing as shown in FIG. 3 will be simply explained. In step S301, the noise reduction unit 445 of the still image selection unit 44 acquires multiple image sets (each including R image data, G image data, and B image data) stored in the storage 42.
In step S302, the noise reduction unit 445 reduces noise of G image data and B image data included in each of the acquired image sets.

In step S303, the contour extraction unit 441 extracts contour components of G image data and B image data subjected to noise reduction at the noise reduction unit 445, for example, by filtering processing.

In step S304, the reduction processing unit 446 performs reduction processing to the contour components of the G image data and B image data extracted by the contour extraction unit 441.

In step S305, the contour expansion processing unit 442 performs contour expansion processing to the contour components of the G image data and B image data subjected to reduction processing at the reduction processing unit 446.

In step S306, the shift amount detector 443 normalizes contour components of R image data, G image data, and B image data subjected to contour expansion processing at the contour expansion processing unit 442.

In step S307, the shift amount detector 443 calculates a shift amount between image data items included in the same image set.
In step S308, the image selection unit 444 selects an image set to be used for still image display, in accordance with the shift amounts between image data items included in each of the image sets.

According to the aforementioned variation example, the shift amount between image data items can be detected with high accuracy, and the possibility of an image set being selected that is not suitable for still image display can be reduced.

**Claims**

**1.** A signal process apparatus comprising:

a contour extraction unit that acquires a plurality of image data items generated by sequential imaging of a subject, and extracts contour components from the acquired plurality of image data items;

a shift amount detector that compares shift amounts of contour components extracted by the contour extraction unit between image data items included in each of a plurality of image sets, each of the image sets having a predetermined number of the image data items, and detects a shift amount between image data items included in each of the image sets; and

an image selection unit that selects at least one image set among the image sets based on a detection result of the shift amount detector.

2. The signal process apparatus according to claim 1, further comprising a storage that stores the plurality of image sets, wherein the image selection unit selects an image set having a smallest shift amount between image data items among the plurality of image sets stored in the storage, based on a still image generation instruction.

3. The signal process apparatus according to claim 1, further comprising a contour expansion processing unit that performs expansion processing to the extracted contour components.

4. The signal process apparatus according to claim 1, wherein the shift amount detector calculates an absolute difference value for each pixel between two image data items among the image data items included in the image set, and detects the shift amount between image data items by a sum of the calculated absolute difference values.

5. The signal process apparatus according to claim 1, further comprising: a noise reduction unit that is provided as a preprocessing stage of the contour extraction unit, and reduces noise components of the image data items.

6. The signal process apparatus according to claim 3, further comprising: a reduction processing unit that is provided as a preprocessing stage of the contour expansion processing unit, and performs reduction processing to the image data items.

7. The signal process apparatus according to claim 6, wherein the reduction processing unit is provided at a preprocessing side of the contour extraction unit.

8. An endoscope system comprising:

an imaging unit that acquires a plurality of image data items by sequential imaging of a subject;
a contour extraction unit that extracts contour components from the plurality of image data items acquired at the imaging unit;
a shift amount detector that compares shift amounts of contour components extracted by the contour extraction unit between image data items included in each of a plurality of image sets, each of the image sets having a predetermined number of the image data items, and detects a shift amount between image data items included in each of the image sets; and
an image selection unit that selects at least one image set among the image sets based on a detection result of the shift amount detector.

F I G. 1

EP 3 207 855 A1

```
                    ┌──────────┐
                    │  Start   │
                    └────┬─────┘
                         │
                         ▼
                    ╱ S101 ╲
              ╱ Is power supply ╲ ──Yes──┐
              ╲  turned off?   ╱         │
                    ╲     ╱               ▼
                     │ No           ┌─────────┐
                     ▼              │   End   │
          ┌───────────────────┐    └─────────┘
          │ Imaging and       │ S102
          │ illuminating      │
          └────────┬──────────┘
                   ▼
          ┌───────────────────┐ S103
          │ Image processing  │
          └────────┬──────────┘
                   ▼
          ┌───────────────────┐ S104
          │  Video display    │
          └────────┬──────────┘
                   ▼
                 ╱ S105 ╲
        No── ╱ Is instruction ╲
             ╲ for still image ╲
              ╲ display indicated? ╱
                   │ Yes
                   ▼
          ┌───────────────────┐ S106
          │ Still image       │
          │ selection         │
          └────────┬──────────┘
                   ▼
          ┌───────────────────┐ S107
          │ Image processing  │
          └────────┬──────────┘
                   ▼
          ┌───────────────────┐ S108
          │ Still image       │
          │ display           │
          └────────┬──────────┘
                   ▼
                 ╱ S109 ╲
           ╱ Is instruction ╲ ──No──┐
           ╲ for still image  ╱      │
            ╲ display        ╱       │
             ╲ termination  ╱        │
              ╲ indicated? ╱         │
                   │ Yes
```

F I G. 2

Start

Input image — S201

Contour extraction processing — S202

Contour expansion processing — S203

Normalization — S204

Calculate shift amount — S205

Image set selection — S206

End

F I G. 3

# FIG.4

Input image 1
(large shift amount)

Input image 2
(medium shift amount)

Input image 3
(no shift amount)

Contour extraction

Contour expansion processing

Calculate absolute difference value after normalization of expansion result

By F1>F2>F3, set input image 3 as optimal still image

EP 3 207 855 A1

44

| Noise reduction unit | Contour extraction unit | Reduction processing unit | Contour expansion processing unit | Shift amount detector | Image selection unit |
|---|---|---|---|---|---|
| 445 | 441 | 446 | 442 | 443 | 444 |

FIG. 5

```
                    ( Start )
                        │
                        ▼
            ┌──────────────────────┐
            │     Input image      │──── S301
            └──────────────────────┘
                        │
                        ▼
            ┌──────────────────────┐
            │   Noise reduction    │──── S302
            └──────────────────────┘
                        │
                        ▼
            ┌──────────────────────────┐
            │ Contour extraction processing │──── S303
            └──────────────────────────┘
                        │
                        ▼
            ┌──────────────────────┐
            │  Reduction processing │──── S304
            └──────────────────────┘
                        │
                        ▼
            ┌──────────────────────────┐
            │ Contour expansion processing │──── S305
            └──────────────────────────┘
                        │
                        ▼
            ┌──────────────────────┐
            │    Normalization     │──── S306
            └──────────────────────┘
                        │
                        ▼
            ┌──────────────────────┐
            │ Calculate shift amount │──── S307
            └──────────────────────┘
                        │
                        ▼
            ┌──────────────────────┐
            │  Image set selection  │──── S308
            └──────────────────────┘
                        │
                        ▼
                    ( End )
```

F I G. 6

## INTERNATIONAL SEARCH REPORT

| International application No. |
| --- |
| PCT/JP2015/077981 |

| A. CLASSIFICATION OF SUBJECT MATTER |
| --- |
| *A61B1/04(2006.01)i, G02B23/24(2006.01)i* |

According to International Patent Classification (IPC) or to both national classification and IPC

| B. FIELDS SEARCHED |
| --- |

Minimum documentation searched (classification system followed by classification symbols)
A61B1/04, G02B23/24

Documentation searched other than minimum documentation to the extent that such documents are included in the fields searched
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>A | JP 10-323326 A (Olympus Optical Co., Ltd.),<br>08 December 1998 (08.12.1998),<br>paragraphs [0037] to [0039], [0044]; fig. 1<br>(Family: none) | 1-2,4-5,8<br>3,6-7 |
| A | JP 2-116350 A (Olympus Optical Co., Ltd.),<br>01 May 1990 (01.05.1990),<br>page 2, upper right column, lines 5 to 18<br>& US 4933758 A<br>column 2, lines 1 to 30 | 1-8 |

| ☐ Further documents are listed in the continuation of Box C. | | ☐ See patent family annex. |
| --- | --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>11 December 2015 (11.12.15) | Date of mailing of the international search report<br>22 December 2015 (22.12.15) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006149483 A